# EUROPEAN PATENT SPECIFICATION

(11) **EP 0 345 415 B1**
(45) Date of publication and mention of the grant of the patent: **30.06.1993**
(21) Application number: 89102547.0
(22) Date of filing: 15.02.1989
(51) Int. Cl.: C12M 3/04, C12M 1/24, B65D 1/02

(54) **Expanded surface roller bottle**
Rollflasche mit vergrösserter Oberfläche
Flacon cylindrique à surface expansée

(30) Priority: 10.06.1988 US 204796
(43) Date of publication of application: 13.12.1989
(73) Proprietor: Becton, Dickinson and Company, Franklin Lakes, New Jersey 07417-1880 (US)
(72) Inventor: Tyndorf, Tadeusz A., Manalapan Township, NJ (US); Chu, I-Hsi Danny, West Orange, NJ (US)
(74) Representative: Selting, Günther, Dipl.-Ing.

(56) References cited:
- EP-A- 0 320 348
- DE-A- 2 062 684
- US-A- 3 853 712
- US-A- 4 289 248
- US-A- 4 317 886

## Description

The present invention relates to a roller bottle for cell growth and production, and more particularly concerns a device having a greatly increased internal surface area of the roller bottle in order to achieve enhanced quantities of cells grown from a single roller bottle. Also, the device is a simplified single bottle produced as a single unitary structure, inexpensively, by blow-molding.

Containers which are used in the laboratory in like situations for culturing of cells are commonly known as "roller bottles". These roller bottles are generally cylindrically shaped and are adapted to rotate about their axes. The internal surfaces of such roller bottles are for providing active surfaces for cells. A liquid growth medium is introduced into the roller bottle. The rotating movement of the bottle keeps the internal surfaces wetted with the liquid medium, thereby encouraging the growth of cells. Rotating rollers in an appropriate apparatus are employed to rotate these roller bottles. Usually, the roller bottle apparatus is adapted to be placed inside an incubator or incubating room to control the temperature of cell growth inside the roller bottles.

It is desirable to grow large amounts of cells, mostly for cell by-products, such as pharmaceutical substances that are secreted by cells; for example, insulin, interferon, urokinase or viral vaccines. The standard roller bottles have been successful in increasing the yield of cell growth in-as-much as the entire inside peripheral surface area can be utilized for cell culturing.

In conceiving ways to increase the yield of growing cells in roller bottles, there are substantial constraints which have to be considered in suggesting improvements. In particular, roller bottle rotation devices are widely used in standard sizes and incubators. These devices are in place in many laboratories and are designed to accept roller bottles of a specific size and shape. Thus, to replace these would be expensive and cause substantial lack of standardization throughout the laboratory field. The outside configuration or diameter of roller bottles is generally not one of the parameters which is changed to improve the yield of cells grown in roller bottles. Accordingly, improvements in roller bottles for increasing cell growth, for practical purposes, is limited to modifications of interior surfaces of the roller bottles, and/or improvements in harvesting the cells once they are grown.

One of the problems with the approaches which have taken place in the past has to do with the fact that involved mechanical improvements to roller bottles increase the cost thereof. Literally, thousands of roller bottles are used on a daily basis and are discarded, once they are used. Therefore, the cost of roller bottles must be decreased because of the vast increase in equipment and development costs for medical applications.

Various approaches have been used in order to increase the surface area internally of roller bottles. One approach is to increase the amount of actual surface available for cells to grow on. Representative of prior art devices which increase the surface area internally of conventional roller bottles are U.S. Patent Number 3,941,661, issued March 2, 1976; and U.S. Patent Number 4,317,886, issued March 2, 1982.

Another approach to increasing the yield of cells developed internally of roller bottles includes the combination of increasing the surface area thereof, and the use of involved mechanical devices cooperating with these increased surfaces in order to remove a greater harvest of cells once they are developed on the increased surface internally of the bottles. Representative of these devices include those taught and claimed in U.S. Patent Number 4,004,981 issued January 25, 1987; U.S. Patent Number 4,065,359 issued December 27, 1977,. and U.S. Patent Number 4,600,694 issued July 15, 1986. While each of the above three patents have the effect of increasing the surface area internally of roller bottles and increasing the yield of cells removed therefrom, the internal device utilized in these patents and the arrangements for scraping the cells from the increased surface areas are very involved, and increase the cost of the individual roller bottles, and the product derived therefrom, substantially. Moreover, these are not single-use devices and have been used largely in the labs where they originated.

EP-A-0 320 348 discloses a roller bottle comprising a wall formed of a plurality of corrugations perpendicular to the axis of the bottle. This document falls within the terms of Article 54(3)EPC and is not relevant to the question of inventive step.

With this invention, by contrast, a roller bottle is provided which is produced as a single one-piece or unitary structure by a simple blow-molding technique. Because of this, the roller bottle of the invention may be mass produced inexpensively. No expensive molding operations are required for forming molds to form roller bottles. Moreover, there are no mechanical devices for insertion into roller bottles once formed, in accordance herewith, or roller bottles which must be assembled by costly labor techniques.

This is achieved by blow-molding roller bottles having pleated or corrugated surfaces extending longitudinally or axially of the roller bottle. The pleats formed in the walls of the roller bottle longitudinally thereof increase the effective surface area internally of the roller bottle within the range of about 60 to 70 percent for providing cell growth area. Because of this, the roller bottles of the invention are produced very inexpensively and supplied at a reasonable cost, used once and then discarded. The roller bottles are configured so that they may be used in conventional laboratory roller bottle apparatus.

In viewing generally the conditions for producing roller bottles in accordance with the invention, a variety of thermoplastic materials may be utilized, including, for example, polystyrene, polyethylene terephthalate, the polyolefins and polyvinyl chloride. Polyethylene terephthalate is particularly desirable because it has been found that cells appear to grow better and in greater numbers on this material.

Other objects and advantages of this invention will be apparent from the following description, the accompanying drawings and the appended claims.

### Description of the Drawings

Fig. 1 is a longitudinal view in elevation of a roller bottle embodying the invention;
Fig. 2 is a sectional view of the device of Fig. 1 taken along lines 2-2 of Fig. 1; and
Fig. 3 is an enlarged partial sectional view of the portion in Fig. 2 designated "A".

### Detailed Description of the Invention

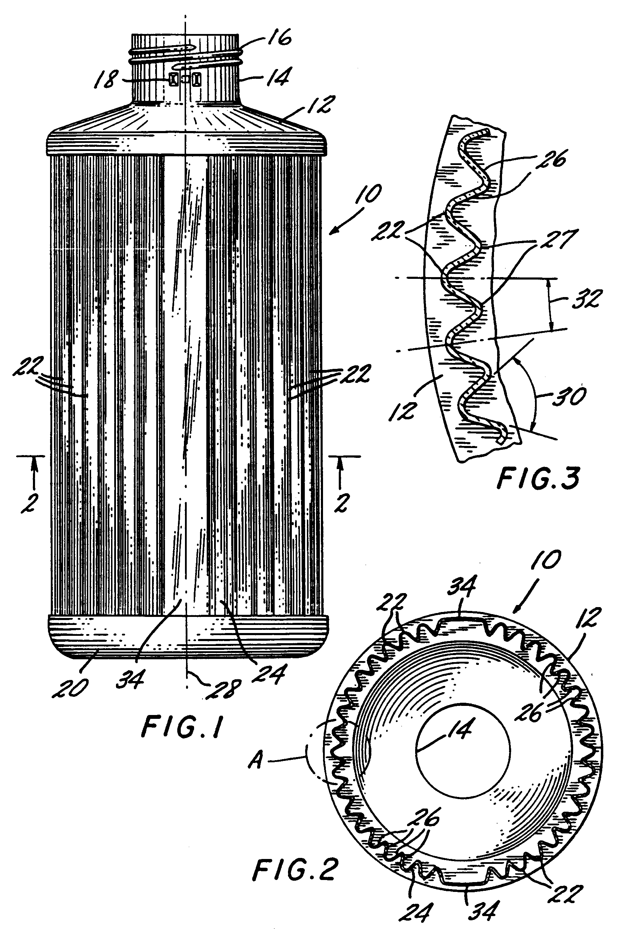

Referring to the drawings in which like reference characters refer to like parts throughout the several views thereof, Fig. 1 shows the roller bottle of the invention generally designated by the reference numeral 10. As can be seen in Fig. 1, roller bottle 10 includes longitudinal pleats 22 extending along the wall 24 of roller bottle 10 in the same direction as axis 28 of roller bottle 10. The tubular wall 24 of roller bottle 10 extends from a base 20 to a top 12. Extending from top 12 and integral therewith is a neck portion 14 having screw threads 16 for receiving a cap thereon in the usual manner. Other cap connections such as a bayonet connection may be used.

Neck 14 may include a locking arrangement 18 for holding a cap in a locked open position on the roller bottle for maintaining the roller bottle open to the environment surrounding it. Such an arrangement is taught and claimed in U.S. Patent Number 4,289,248.

Roller bottle 10 may have diametrically opposed sections 34 where no pleats 22 are included in order to enhance the microscopic viewing of the contents of roller bottle 10 and/or to facilitate the formation thereof.

Referring now to Fig. 2, the sectional view of roller bottle 10 shows the individual pleats or corrugations 22 in wall 24 of roller bottle 10. As can be seen in Fig. 2, the individual pleats or corrugations 22 provide a plurality of opposed surfaces 26 for the formation of cell growth thereon. It is clear that this pleated structure increases the active surface area internally of roller bottle 10 as opposed to a conventional flat wall structure.

Referring now to Fig. 3, a detailed sectional view of the individual pleats 22 are shown. Representative dimensions and angles for a roller bottle produced according to the invention include angle 30 which may be, for example, 60°, while angle 32 may be 9°. The distance between the top 27 of two pleats 22 forming the internal surfaces 26 may be, for example, 0.82 cm (0.323 inches). It should be understood that these dimensions are being disclosed as representative only, and not as a fixed limitation on the disclosure herein. These dimensions are for a roller bottle which is around 27.08 cm (10.66 inches) in length from the top of neck 14 to the bottom surface of base 20. With such a roller bottle dimension, the growth surface area length is about 22.86 cm (9.0 inches), while the diameter of such a roller bottle is about 11.76 cm (4.63 inches).

After the usual formation of cells with a roller bottle 10, in accordance herewith, rolling in the proper environment for the formation of the cells, the procedure utilized with the invention herein is to remove the roller bottle with the formed cells on the walls thereof from the conventional roller apparatus. The cell forming liquid media remaining in the roller bottle may be decanted from the bottle and a small amount of saline solu tion added to prevent the cells from "drying." Alternatively, the liquid media may remain if it is only a small amount. Thereafter, the procedure may be to utilize a scraper apparatus such as that described in co-pending application Serial Number 143,480, filed January 13, 1988 (US-A-4 810 652), for removing cells from the internal surface.

A more conventional procedure for removing cells is the introduction of a conventional solution of a proteolytic enzyme, such as Trypsin, together with a chelating agent, which has the effect of causing the cells to release from the internal surface for decanting from the roller bottle. Subsequently, the bottle is discarded.

Thus, as will be appreciated from the above, there is provided in accordance with this invention, a simplified one-piece unitary roller bottle with greatly increased surface area for cell growth formation therein. The roller bottle is of a configuration which allows for inexpensive production thereof by blow-molding techniques. Thus, the roller bottle of the invention may be produced in large quantities inexpensively, used once and discarded. No expensive procedures are involved for assembling the bottle of the invention and no expensive molds are required for the formation thereof.

For example, roller bottles of different lengths may be produced, in accordance with roller bottle apparatus requirements already in existence. These modifications may be made without any expensive reassembly provisions in order to produce the roller bottles. Thus, mass production techniques may be used in a simplified form to produce many roller bottles of the kind to which the invention is directed.

## Claims

1. A roller bottle for cell growth culturing, characterized by
(a) a substantially cylindrical plastic housing capable of being blow molded;
(b) said housing being comprised of a single unitary structure defining a hollow chamber within;
(c) said housing being closed at one end, and having a liquid opening at the end opposite said closed end;
(d) said liquid opening being substantially on the longitudinal axis of said housing;
(e) said substantially cylindrical housing being comprised of a plurality of pleats therearound;
(f) said pleats extending longitudinally of said housing from said closed end to said liquid opening, and
(g) the interior surface of said pleats adapted to grow cells thereon.

2. The roller bottle of Claim 1, further characterized by
(a) the end of said housing having said liquid opening includes an integral top;
(b) said integral top having a neck integral therewith, said neck defining said liquid opening.

3. The roller bottle of Claim 2, further characterized by (a) said neck including integral means thereon for receiving a cap for closing said liquid opening.

4. The roller bottle of Claim 3, further characterized by
(a) said neck including integral locking means for holding a cap in an open locked position on said neck.

5. The roller bottle of Claim 1, further characterized by
(a) said unitary housing being comprised of a member selected from the group consisting of polystyrene, polyolefin, polyethylene terephthalate, polyvinyl chloride and mixtures thereof.

## Patentansprüche

1. Rollflasche für Zellwachstumskulturen, gekennzeichnet durch
(a) ein im wesentlichen zylindrisches Kunststoffgehäuse, das blasformbar ist;
(b) wobei das Gehäuse aus einer einzelnen einstückigen Struktur besteht, die in sich eine hohle Kammer begrenzt;
(c) wobei das Gehäuse an einem Ende geschlossen ist und an dem dem geschlossenen Ende gegenüberliegenden Ende eine Flüssigkeitsöffnung aufweist;
(d) wobei sich die Flüssigkeitsöffnung im wesentlichen auf der Längsachse des Gehäuses befindet;
(e) wobei das im wesentlichen zylindrische Gehäuse aus mehreren um dieses herum vorgesehenen Falten besteht;
(f) wobei sich die Falten in Längsrichtung des Gehäuses vom geschlossenen Ende zur Flüssigkeitsöffnung erstrekken, und
(g) wobei die Innenfläche der Falten zum Aufwachsenlassen von Zellen darauf geeignet ist.

2. Rollflasche nach Anspruch 1, ferner dadurch gekennzeichnet, daß
(a) das mit der Flüssigkeitsöffnung versehene Ende des Gehäuses einen damit einstückigen oberen Bereich aufweist;
(b) wobei der einstückige obere Bereich einen mit diesem einstückigen Hals aufweist, der die Flüssigkeitsöffnung begrenzt.

3. Rollflasche nach Anspruch 2, ferner dadurch gekennzeichnet, daß
(a) der Hals auf diesem einstückig vorgesehene Einrichtungen zur Aufnahme einer Kappe zum Verschließen der Flüssigkeitsöffnung aufweist.

4. Rollflasche nach Anspruch 3, ferner dadurch gekennzeichnet, daß
(a) der Hals einstückige Verriegelungseinrichtungen zum Halten der Kappe in einer geöffneten Verriegelungsposition auf dem Hals aufweist.

5. Rollflasche nach Anspruch 1, ferner dadurch gekennzeichnet, daß
(a) das einstückige Gehäuse aus einem Material besteht, das aus der Gruppe ausgewählt ist, die Polystyrol, Polyolefin, Polyethylenterephthalat, Polyvinylchlorid und Mischungen aus diesen enthält.

## Revendications

1. Bouteille roulante pour la culture de cellules, caractérisée par :
(a) un boîtier de plastique sensiblement cylindrique susceptible d'être moulé par soufflage ;
(b) ledit boîtier étant constitué d'une structure unitaire unique dont l'intérieur définit une chambre creuse ;
(c) ledit boîtier étant fermé à une extrémité et ayant une ouverture à liquide à l'extrémité opposée de ladite extrémité fermée ;
(d) ladite ouverture à liquide étant située sensiblement sur l'axe longitudinal dudit boîtier ;
(e) ledit boîtier sensiblement cylindrique étant constitué d'une série de plis distribués tout autour ;
(f) lesdits plis s'étendant longitudinalement dans ledit boîtier de ladite extrémité fermée à ladite ouverture à liquide ; et
(g) la surface interne desdits plis étant à même de faire croître des cellules.

2. Bouteille roulante selon la revendication 1, caractérisée en outre en ce que :
(a) l'extrémité dudit boîtier portant ladite ouverture à liquide comporte un couvercle qui en fait partie intégrante ;
(b) ledit couvercle présentant un col intégré qui définit ladite ouverture à liquide.

3. Bouteille roulante selon la revendication 2, caractérisée en outre en ce que :
(a) ledit col porte des moyens intégrés pour recevoir un bouchon afin de fermer ladite ouverture à liquide.

4. Bouteille roulante selon la revendication 3, caractérisée en outre en ce que :
(a) ledit col comprend des moyens de verrouillage qui en font partie intégrante pour maintenir un bouchon en position d'ouverture bloquée sur ledit col.

5. Bouteille roulante selon la revendication 1, caractérisée en outre en ce que :
(a) ledit boitier unitaire est constitué d'un élément choisi parmi le polystyrène, les polyoléfines, le téréphtalate de polyéthylène, le chlorure de polyvinyle et leurs mélanges.
